# EUROPEAN PATENT APPLICATION

(11) **EP 1 538 205 A1**
(43) Date of publication of application: **08.06.2005**
(21) Application number: 03078861.6
(22) Date of filing: 05.12.2003
(51) Int. Cl.: C12N 9/84, C12P 35/02, C12N 15/63, C12N 15/10

(54) **Glutaryl amidases and their uses**

(71) Applicant: STICHTING VOOR DE TECHNISCHE WETENSCHAPPEN, 3527 JP Utrecht (NL)
(72) Inventor: Sio, Charles Fredrik, 9712 GT Groningen (NL); Cool, Robbert Hans, 9722 WN Groningen (NL); Otten, Ludwina Gerharda, 7921 TH Zuidwolde (NL); Quax, Wilhelmus Johannes, 9606 PT Kropswolde (NL)
(74) Representative: Prins, Adrianus Willem, Mr. Ir.

(57) **Abstract**

The invention relates to a new class of isolated, single or multiple mutated glutaryl amidases from *Pseudomonas* SY-77, or a functional part, derivative or analogue thereof. The invention also relates to a method for preparing 7-aminocephalosporanic acid from cephalosporin C, using a glutaryl amidase according to the invention. The invention also provides a method for preparing aminodesacetoxycephalosporanic acid.

## Description

The present invention relates to a new class of glutaryl amidases and variants and their uses, in particular their use in a process for the preparation of 7-aminocephalosporanic acid (7-ACA).

It is known that 7-ACA can be used as the starting product for the semi-synthetic preparation of various cephalosporin antibiotics, and that there exist various processes to prepare 7-ACA on a large scale. Among these known processes the enzymatic processes are preferred from both economic and environmental perspective.

In one of the known production processes, cephalosporin C is decarboxylated by chemical oxidation to form glutaryl-7-ACA, and the so obtained substrate is subsequently processed to form 7-ACA using the enzyme glutaryl amidase. This process has, however, the disadvantage that it involves a high level of environmental pollution, resulting in high costs.

In another known production process (as for example described in W003076640), cephalosporin C is firstly subjected to an oxidative decarboxylation treatment which is carried out enzymatically with the help of D-amino acid oxidase. The glutaryl-7-ACA so obtained is then converted into 7-ACA using the enzyme glutaryl amidase. Although the latter enzymatic process is already much more attractive than the former it still involves several process steps and therefore there is still significant room to develop an improved process for the conversion of cephalosporin C into 7-ACA.

In view of the commercial interest of such an application, many attempts have been made to isolate an amidase that can hydrolyse cephalosporin C to produce 7-ACA. Enzymes from different organisms have been isolated and tested on their capability to hydrolyse cephalosporin C. Examples can be found in patents WO0224879, US5229274, EP-A-0283218, EP-A-0322032, and US4774179. Nevertheless, so far the cephalosporin C hydrolysis activity in any of the known amidases is too low for commercial interest.

Also attempts have been made to mutate glutaryl amidase at specific positions in order to increase the cephalosporin C hydrolysis activity. For example, the glutaryl amidase as described in US4774179 was mutated at several positions, but so far no commercially usable variants have been obtained [ref. 1-4]. Further in WO 02/072806 mutations in another glutaryl amidase have been proposed on the basis of structural modelling, without any experimental substantiation [ref. 5]. In yet another approach, mutations were introduced in a random manner, after which variants with an activity towards a desired substrate were selected. As an example of the latter, the glutaryl amidase of *Pseudomonas* SY-77 was mutated and variants of the amidase with the desired activity were isolated by growth on selective medium. Several mutants, amongst which a variant carrying the mutation Tyr178 into His, were claimed (US5457032). The latter mutant was shown to have increased hydrolysis activity on adipyl-7-aminodesacetoxycephalosporanic acid (7-ADCA; ref. 6). This compound is, like 7-ACA, an industrially important reagent for the synthesis of semi-synthetic cephalosporin antibiotics [ref. 7]. However, in spite of the fact that this mutant was claimed more than ten years ago, no activity for cephalosporin C was demonstrated so far. It is further observed that mutations at other positions in *Pseudomonas diminuta* cephalosporin acylase were predicted by modelling studies, and claimed to increase the cephalosporin C hydrolysis activity of a glutaryl amidase several fold [ref. 8]. However, no formation of 7-ACA was shown. Only a few variants were predicted and there was clearly no teaching for further improvement by making, for instance, combinations of different mutations.

In order to isolate new mutants of *Pseudomonas* SY-77 with increased hydrolysis activity towards adipyl-7-ADCA, an unbiased approach using mutagenesis and growth selection on solid medium containing adipyl-leucine was used recently [ref. 6, 9]. However, no activity on cephalosporin C was shown.

It has now surprisingly been found that a very attractive single-enzyme process can be developed for the production of 7-ACA from cephalosporin C when use is made of a newly developed class of glutaryl amidases.

Accordingly, one aspect of the present invention relates to an isolated or single mutated glutaryl amidase from *Pseudomonas* SY-77, or a functional part, derivative or analogue thereof, having an amino acid at position 266 other than asparagine, histidine or serine.

With the term "single mutated" is meant that the glutaryl amidase is derived from a naturally occurring glutaryl amidase that has only been mutated in one position. In other words, Asn266 has been mutated into an amino acid other than asparagine.

A major advantage of this newly developed class of glutaryl amidases is that they enable the direct conversion of cephalosporin C into 7-ACA in a much simpler and cheaper way. Further, these variants can suitably be used to prepare 7-ACA on an industrial scale. In such processes, the glutaryl amidase according to the present invention binds cephalosporin C as a substrate and catalyses the conversion of the cephalosporin C into 7-ACA, whereby the amino acid at position 266 directly or indirectly increases the cephalosporin C hydrolysis activity of said amidase.

Preferably, the present invention relates to an isolated or single mutated glutaryl amidase from *Pseudomonas* SY-77, or a functional part, derivative or analogue thereof, having a glutamine, methionine or tryptophan at position 266. More preferably such glutaryl amidase has a glutamine or methionine at position 266. Most preferably, such glutaryl amidase has a glutamine at position 266.

Another aspect of the present invention is that the effect of a mutation in position 266 on cephalosporin C hydrolysis activity can be improved by the additional presence of other mutations in or near to the substrate binding pocket. Amino acids that directly or indirectly contribute to substrate binding, or that may otherwise contribute to substrate specificity, are found in positions: Leu177, Tyr178, Val179, His221, Leu222, Phe229, Tyr231, Gln248, Arg255, Phe256, Thr267, Val268, Met271, Gln291, Met347, Tyr351, Thr374, Phe375 and/or Asn442. It can not be excluded that variants of glutaryl amidase with mutations in these positions have a high cephalosporin C hydrolysis activity even in combination with Asn266.

Accordingly, another attractive aspect of the invention relates to an isolated or multiple mutated glutaryl amidase from *Pseudomonas* SY-77, or a functional part, derivative or analogue thereof, having an amino acid other than its native amino acid at position Asn266, and having an amino acid other than its native amino acid at one or more of the following positions: Leu177, Tyr178, Val179, His221, Leu222, Phe229, Tyr231, Gln248, Arg255, Phe256, Thr267, Va1268, Met271, Gln291, Met347, Tyr351, Thr374, Phe375 and/or Asn442.

With the term "multiple mutated" is meant that the glutaryl amidase is derived from a naturally occurring glutaryl amidase that has been mutated in at least two positions. In other words, Asn266 has been mutated into another amino acid, whereas in addition one or more other native amino acids have been mutated into other amino acids.

As mentioned hereinabove, these glutaryl amidases display an improved efficiency in the preparation of 7-ACA from cephalosporin C.

Preferably, the isolated or multiple mutated glutaryl amidase according to the invention has in addition to an amino acid other than asparagine at position 266, one or more mutations at the following positions: Tyr178, Leu222, Tyr231, Gln248, Arg255, Val268, Tyr351 and/or Phe375.

Most preferably, the isolated or multiple mutated glutaryl amidase comprises in addition to an amino acid other than asparagine at position 266, one or more mutations at the following positions:
Tyr178,
Tyr231,
Arg255,
Phe375,
Tyr178 + Tyr231,
Tyr178 + Arg255,
Tyr178 + Phe375,
Tyr231 + Arg255,
Tyr231 + Phe375,
Tyr231 + Arg255 + Phe375,
Arg255 + Phe375,
Tyr178 + Tyr231 + Arg255,
Tyr178 + Tyr231 + Phe375,
Tyr178 + Arg255 + Phe375, or
Tyr178 + Tyr231 + Arg255 + Phe375.

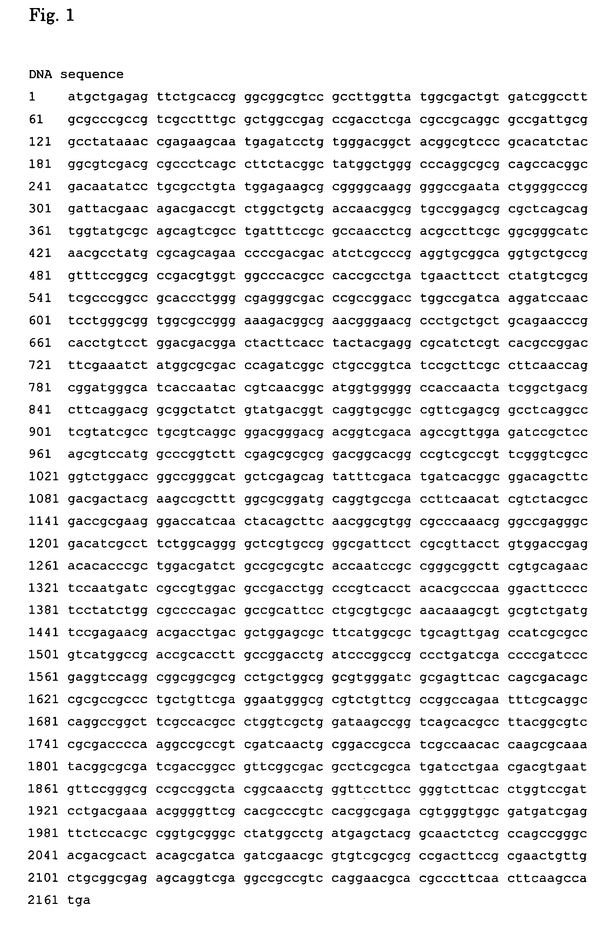

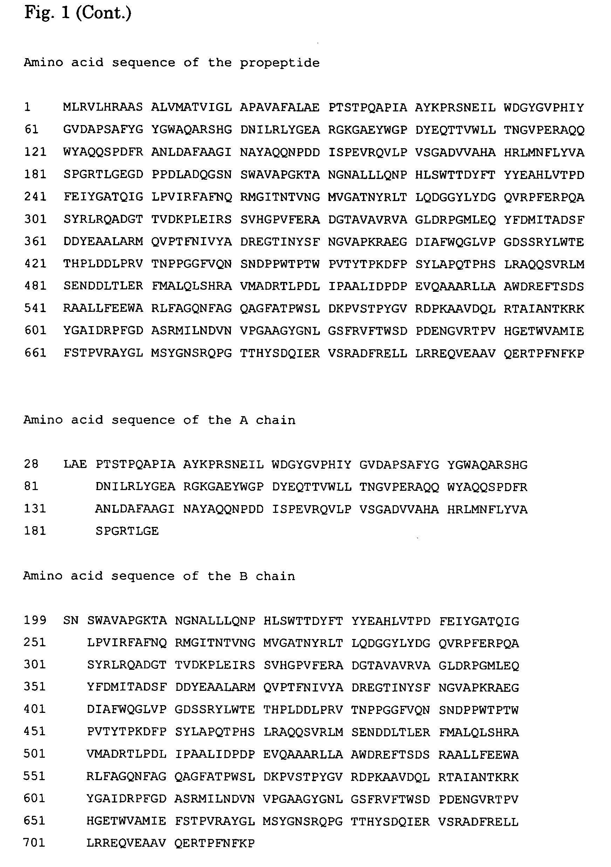

All amino acid numbering in the present application is with respect to the propeptide chain as shown in fig. 1. The numbering applies to equivalent positions in amino acid sequences of other glutaryl amidases as determined for example by sequence alignment.

In the context of the present invention, a mutation of a position in the glutaryl amidase implies the incorporation of any of the 20 natural amino acids other than the native amino acids at that particular position in the glutaryl amidase (as listed in fig. 1), or the incorporation of a non-natural amino acid at that particular position or a modification of the amino acid at that particular position.

The glutaryl amidase is obtained from a *Pseudomonas* SY-77, or is a functional part, derivative or analogue thereof. In the context of the present application, derivates or analogues of said *Pseudomonas* SY-77 glutaryl amidase include: *Pseudomonas* C427 glutaryl amidase [ref. 10], *Pseudomonas* sp. 130 glutaryl amidase [ref. 11,12], *Pseudomonas* GK-16 glutaryl amidase [ref. 13,14], and *Pseudomonas diminuta* KAC-1 glutaryl amidase [ref. 15,16].

The glutaryl amidase of *Pseudomonas* SY-77 has, for instance, been described in references [ref. 6,17]. The homologues of *Pseudomonas* SY-77 glutaryl amidase as used in accordance of the present invention have, for instance, been described in references [ref. 10-16].

The present invention also provides a polynucleotide encoding a variant of a glutaryl amidase as described hereinabove.

The present invention further provides a nucleic acid vector comprising a polynucleotide encoding a variant of a glutaryl amidase as described hereinabove.

Additionally, the present invention provides a host cell comprising a polynucleotide encoding a variant of a glutaryl amidase as described hereinabove.

The present invention also relates to a method for producing any one of the glutaryl amidases according to the present invention, which method comprises culturing the hereinabove-mentioned host cell under suitable conditions which allow for expression of the glutaryl amidase in said host cell.

In the context of the invention, variants of the glutaryl amidase will be produced in host cells by expression of the encoding gene or by production in a cell-free system. Host cells principally can be prokaryotic or eukaryotic cells, but are by preference prokaryotic. More preferably, the host cells are bacterial cells such as *Escherichia coli* or *Bacillus subtilis* or *Pseudomonas.* Examples of such expression systems can be found in ref. 6 and 18. A cell-free system, such as the Rapid Translation System (Roche) can also be used to produce said variants. Alternatively, both subunits of the glutaryl amidase can be co-expressed, as for example described in ref. 11.

By way of an example, recombinant *E. coli* clones expressing the variant of a glutaryl amidase under control of a strong promoter according to the present invention is typically cultured to a 0.1-10 litre scale. The cells are initially concentrated by centrifugation. Subsequently, they are resuspended in a small volume of a suitable buffer and disrupted. After centrifugation to remove the cell debris, the supernatant is used in a purification strategy to obtain the purified enzyme.

By way of an example, and more suitable for smaller quantities, the cells may be lysed by sonication, followed by a three-step purification using different chromatographic steps as described hereinbelow in Example 1.

Alternatively, and more suitable for an industrial scale, cells may be resuspended in 100 mM phosphate buffer at a pH of 7.2, and disrupted by means of a French press. To remove the cell debris, the suspension is then centrifuged at 10,000 rpm for 15 minutes, and the supernatant is transferred to another tube. The enzyme-containing supernatant solution is then rebuffered in an Amicon ultrafiltration cell or by diafiltration against 0.8-1.5 M, more preferably in the range of from 1-1.5 M, potassium phosphate buffer at a pH of 7.8 at 4°C with simultaneous concentration. If the concentrated solution is left to stand in cold conditions, the enzyme crystallises out. The crystals may be enriched by gentle centrifugation and washed with the above-mentioned buffer. In this way large amounts of purified enzyme can be obtained in a simple, swift and cheap manner, without using expensive chromatographic purification steps.

In a preferred embodiment, cephalosporin C hydrolysis is carried out at a pH in the range of from pH 7.5 to 8.5, more preferably in the range of from 7.5 to 8.2. In that way, decomposition of cephalosporin C is minimised.

The concentration of cephalosporin C in the above method lies preferably in the range from 20-200 mM, more preferably in the range of from 50 to 120 mM.

In the above method, the variant of the glutaryl amidase is suitably used in a concentration in the range of from 0.25 to 10 mg/ml in the reaction mixture, and preferably in the range of from 1 to 4 mg/ml.

The above method is suitably carried out at a temperature in the range of from 15 to 45°C, preferably in the range of from 25 to 40°C.

The reaction time for cephalosporin C hydrolysis is suitably in the range of from 1 to 40 hour, preferably in the range of from 1 to 20 hour.

The variants of a glutaryl amidase according to the present invention can also be applied carrier-fixed. In this way, a further reduction of costs can be established. Suitable carrier materials that can be used for this purpose have for instance been described in ref. 19. Alternatively, variants of a glutaryl amidase according to the present invention can also be applied as carrier-free cross-linked enzymes as for instance described in Cao *et al.* [ref. 20].

The variants of a glutaryl amidase according to the present invention can be used to produce 7-ACA directly from cephalosporin C.

Accordingly, the present invention also provides a method for preparing 7-ACA from cephalosporin C, which method comprises reacting cephalosporin C with an isolated, single mutated or multiple mutated glutaryl amidase from *Pseudomonas* SY-77, or a functional part, derivative or analogue thereof, having an amino acid at position 266 other than asparagine under suitable conditions such that the glutaryl amidase cleaves the cephalosporin C directly to form the 7-aminocephalosporanic acid.

In said method any of the glutaryl amidases according to the present invention can suitably be used.

Preferably, the present invention relates to a method for preparing 7-ACA from cephalosporin C, which method comprises reacting cephalosporin C with a glutaryl amidase under suitable conditions such that the variant of a glutaryl amidase cleaves the cephalosporin C directly to form the 7-ACA, wherein the glutaryl amidase comprises an isolated or multiple mutated glutaryl amidase from *Pseudomonas* SY-77, or a functional part, derivative or analogue thereof, having an amino acid other than its native amino acid at position Asn266, and having an amino acid other than its native amino acid at one or more of the positions Leu177, Tyr178, Val179, His221, Leu222, Phe229, Tyr231, Gln248, Arg255, Phe256, Thr267, Va1268, Met271, Gln291, Met347, Tyr351, Thr374, Phe375 and/or Asn442.

Surprisingly, it has also been found that the mutation of Tyr178 into another amino acid in said *Pseudomonas* SY-77 glutaryl amidase increases hydrolysis activity on cephalosporin C (see table 2). This particular mutant has been isolated (ref. 6) but its unexpectedly high activity on cephalosporin C was not known before.

Accordingly, the present invention also relates to a method for preparing 7-aminocephalosporanic acid from cephalosporin C, which method comprises reacting cephalosporin C with an isolated, single or multiple mutated glutaryl amidase from *Pseudomonas* SY-77, or a functional part, derivative or analogue thereof, having an amino acid other than its native amino acid at position Tyr178, under suitable conditions such that the glutaryl amidase cleaves the cephalosporin C directly to form the 7-aminocephalosporanic acid.

Preferably, the said glutaryl amidase is a single mutated glutaryl amidase.

More preferably, the amino acid at position Tyr178 of the said glutaryl amidase is a histidine.

It has further surprisingly been found that some of the single mutants that were isolated have a high hydrolysis activity in respect of the production of adipyl-7-aminodesacetoxycephalosporanic acid (7-ADCA). This compound is, like 7-ACA, an industrially important reagent for the synthesis of semi-synthetic antibiotics.

The present invention therefore also relates to a method for preparing 7-ADCA, which comprises reacting adipyl-7-ADCA with an isolated or single mutated glutaryl amidase from *Pseudomonas* SY-77, or a functional part, derivative or analogue thereof, having an amino acid other than its native amino acid at position Asn266, under suitable conditions such that the glutaryl amidase cleaves adipyl-7-ADCA to form the 7-ADCA. Preferably, the amino acid at position 266 of said glutaryl amidase is a glutamine, methionine, phenylalanine, tryptophan or tyrosine. More preferably, the amino acid is a methionine or glutamine.

It will be appreciated by the person skilled in the art that the said variants with increased hydrolysis activity to produce 7-ACA or 7-ADCA can also be used for the synthesis of new cephalosporin antibiotics from these compounds [ref. 21]. Hence, the present invention also relates to a method for preparing 7-ACA based antibiotics comprising incubating 7-ACA in the presence of the glutaryl amidase according to the present invention with alkyl side chains under suitable synthesizing conditions.

The glutaryl amidases according to the present invention consist of two sub-units of different sizes which are formed from a propeptide comprising amino acids through processing steps taking place in the periplasm of the expression organism to form a functional enzyme. The small sub-unit (A chain) comprises 161 amino acids in the functional enzyme, the large sub-unit (B chain) 522 amino acids. The protein sequences of the A and B chains are shown in the sequence listing part of the description respectively. Numbering of residues throughout is with reference to the amino acid sequences. However, it will be understood that where other glutaryl amidases are used, the numbering applies to the functionally corresponding residues in those sequences that do not have exactly the same numbering as in the sequence listing part. Furthermore, it will be understood by a person skilled in the art that functional parts of different sizes of the said glutaryl amidase or derivative or analogue can be derived without significantly affecting hydrolysis activity.

Mutations in the glutaryl amidase gene sequence may be introduced, for example, using standard site directed mutagenesis techniques, such as those described in ref. 22.

As mentioned earlier, the present invention also provides a polynucleotide encoding a variant of a glutaryl amidase according to the present invention. Such polynucleotide may comprise DNA or RNA. The polynucleotide may include synthetic or modified nucleotides. Various methods to modify oligonucleotides are known in the art. These methods include the use of methylphosphonate and phosphorothioate backbones, with addition of acridine or polylysine chains at the 3' and/or 5' ends of the molecule. The polynucleotides as described hereinabove may be modified by any method available in the art. It will be appreciated by the skilled person in the art that various different polynucleotides can encode the same polypeptide as a result of the degeneracy of the genetic code.

Another aspect of the invention relates to a method for developing a variant of a cephalosporin amidase with high cephalosporin C hydrolysis activity. In this method use is made of a particular combination of techniques.

Accordingly, the present invention also relates to a method for developing a glutaryl amidase with cephalosporin C hydrolysis activity comprising the steps of:
1) applying a mutagenesis technique in order to create a large library of mutants of a glutaryl amidase according to the present invention that carries a mutation in one or more positions; and subsequently
2) applying a selection procedure comprising growth of proper host cells that express variants of said glutaryl amidase on solid medium containing a selective, aminoadipyl-containing compound and identification of variants with the desired enzymatic activity.
This process enables the identification and preparation of cephalosporin amidases that display a high cephalosporin C hydrolysis activity.

The combination of techniques as indicated hereabove can be applied to *Pseudomonas* SY-77, or a functional part, derivative or analogue thereof.

The present invention will now be shown by means of the following Examples, which are non-limiting and have been included for illustration purposes only.

### EXAMPLES

### Example 1 - Preparation and purification of Pseudomonas SY-77 glutaryl amidase mutants modified at position 266

The megaprimer method [ref. 23] was used in order to introduce the 19 amino acid other than asparagine on position 266. First, the plasmid pMcSY-2 comprising the wild-type (WT) sequence for *Pseudomonas* SY-77 glutaryl amidase and a primer with a randomised codon 266 was used in a PCR reaction. The PCR fragment was digested with restriction endonucleases *BglII* and *Sst*II, and cloned into vector pMcSY-2 digested with the same endonucleases by ligation. Plasmid DNA of a total of 96 transformants was sequenced. This resulted in the isolation of mutants carrying 17 different amino acids at this position in a single mutagenesis round. The missing 3 mutants were made by site-directed mutagenesis using specific primers. *Escherichia coli* DH10B cells containing the plasmid encoding the (mutant) enzyme were grown in 300 ml 2*YT-medium at 25°C. After 30 hours the cells were harvested by centrifugation. A cell-free extract was made by sonication (10 min, output 4, 50% duty cycle on a Sonifier 250, Branson) and centrifugation (30 min at 14000 rpm). This extract was used to purify the enzyme in a 3-step protocol on a Duoflow system (Bio-Rad) using columns from Amersham Pharmacia. The cell free extract was loaded onto a HiTrapQ column and the protein eluted with a linear gradient of 0-1 M NaCl in 50 mM Tris-HCl pH 8.8. After analysis on SDS-PAGE, the fractions containing the enzyme were loaded onto a HiTrap PhenylSepharose HP column. The protein was eluted with a gradient of 0.7-0 M (NH₄)₂SO₄. The pooled fractions containing the enzyme were desalted on a HiPrep 26/10 Desalting column, and reloaded onto a HiTrapQ column. After rinsing with 220 mM NaCl, the protein was eluted with 330 mM NaCl. The pooled fractions were stored at -20°C. Typical yields were 10 mg of more than 90 % pure enzyme per liter of culture.

### Example 2 - Conversion of cephalosporin C into 7-aminocephalosporanic acid

For the determination of activity on cephalosporin C, 70 µg of various SY-77 glutaryl amidase variants and wild-type enzymes were incubated for 5 hours at 37°C with 10 mM cephalosporin C in 300 µl of phosphate buffer (20M, pH 7.5). Aliquots of 40 µl were taken from the reaction mixture every hour and transferred to 140 µl of 0.5 M acetate buffer pH 4.5. 20 µl of a 1 mg/ml fluorescamine in acetone solution was added, and the absorbance at 380 nm was measured on a spectrophotometer after 1 hour incubation. The product of the deacylation reaction, 7-aminocephalosporanic acid, forms a complex with fluorescamine that absorbs light of 380 nm wavelength [ref. 24]. The mean slope of the conversion of cephalosporin C in the first 4 hours was determined and normalised towards WT.

### Example 3 - Construction of a library of glutaryl amidase mutants randomised at positions 266 and 375

A pool of vectors harboring glutaryl amidase genes with 20 different codons encoding all 20 naturally occurring amino acids at position 375 was digested with restriction endonucleases *Nco*I and *HindI*II, and the small fragment was ligated into the large fragments of a pool of vectors harboring glutaryl amidase genes with 20 different codons encoding all 20 naturally occurring amino acids at position 266 digested with the same restriction endonucleases. This pool of vectors harboring glutaryl amidase genes with altered codons encoding the amino acids at positions 266 and 375 was transformed to *E*. *coli* DH10B cells, resulting in a mutant library in which positions 266 and 375 were completely randomized.

### Example 4 - Construction of a library of glutaryl amidase mutants randomized at positions 178, 231, 255, 266 and 375

A mutant library of glutaryl amidase genes, which are randomized in five codons (encoding amino acids at positions 178, 231, 255, 266 and 375), was made by the megaprimer method as described by Landt *et al* [ref. 23]. Primers according to parts of the glutaryl amidase gene, but with randomized codons encoding amino acids at position 178, 231, and 255+266 were used to amplify the section containing the codons encoding these 4 positions of the glutaryl amidase gene. The nucleotide sequences of these primers are listed in table 1. The library was constructed in four steps:

| | Forward primer | Backward primer | Product |
|---|---|---|---|
| Step 1. | F1 | B1 | P1 |
| Step 2. | F2 | P1 | P2 |
| Step 3. | P2 | B2 | P3 |
| Step 4. | P3 | B3 | P4 |

This product P4 was digested with EcoO190I and NcoI, and ligated into the large fragments of a pool of vectors harboring glutaryl amidase genes with 20 different codons encoding all 20 amino acids at position 375 digested with the same restriction endonucleases. This pool of vectors harboring glutaryl amidase genes with altered amino acids at positions 178, 231, 255, 266 and 375 was transformed to *E. coli* DH10B cells, resulting in a mutant library in which positions 178, 231, 255, 266 and 375 were completely randomized.

### Example 5 - Selection of variants of Pseudomonas SY-77 on hydrolysis activity for aminoadipyl-leucine or other aminoadipvl-amino acids

A library as described in example 4 was selected on minimal medium containing aminoadipyl-leucine as sole leucine source. 10⁷ different colonies were starved by incubation in 0.9% NaCl for 2 h at 37°C and plated onto selective minimal medium plates containing 0.1 mg/ml aminoadipyl-leucine as a sole leucine source. Approximately 10000 viable *E*. *coli* DH10B cells containing the mutant plasmids were spread onto each plate. Plates were incubated at 30°C for 6 days. Every day new appearing colonies were marked. Colonies were selected on the basis of their date of appearance and size. 57 colonies were picked and grown overnight at 30°C. From each culture the plasmid was isolated and the presence of glutaryl amidase was detected using a polyclonal antibody against the glutaryl amidase of *Pseudomonas* SY-77. Plasmid were retransformed to *E. coli* DH10B cells and streaked again on minimal medium containing adipyl-leucine as sole leucine source to ensure unique colonies were obtained. Single colonies from these second plates should be used to inoculate an overnight culture to determine acylase activity and to isolate plasmid DNA for sequence determination.

### Example 6 - Some single mutated variants of Pseudomonas SY-77 glutaryl amidase with increased cephalosporin C hydrolysis activity

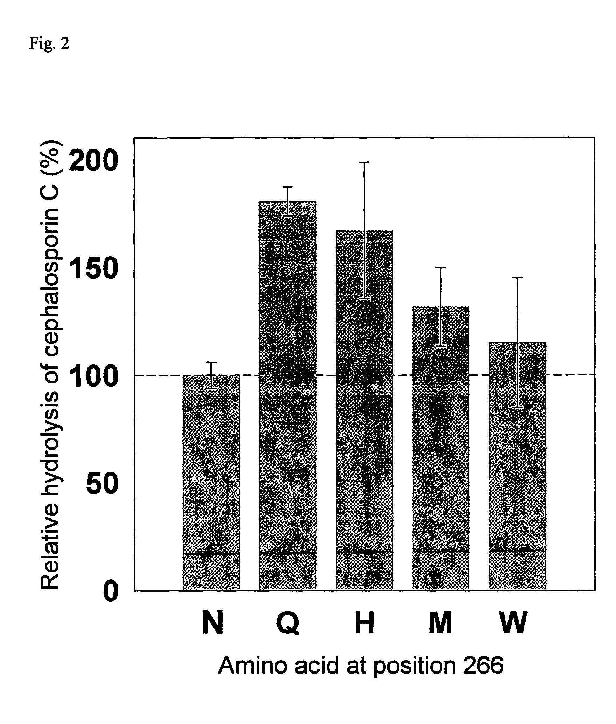

Wild-type (WT) and 15 glutaryl amidases variants mutated at position 266 were produced in *E. coli* DH10B and purified up to 95% purity. The 4 glutaryl amidases comprising single mutations N266R, N266I, N266K and N266V could not be produced. 20 µg of the mutant and WT enzymes were incubated at 37°C with 10 mM cephalosporin C. The WT enzyme and the variants carrying mutants Asn266 into Gln, Asn266 into His, Asn266 into Met or Asn266 into Trp showed some hydrolysis of the cephalosporin C after 20 hours. These activities were too low to be able to determine Kₘ and k_{cat}. In order to avoid long incubation times that may lead to enzyme destabilisation, the conversion rate of the different mutants was calculated from an assay with more enzyme (70 µg) as given in example 2. The mean slope of the conversion of cephalosporin C in the first 4 hours was determined and normalised towards WT. All the tested variants, carrying mutants Asn266 into Gln, Asn266 into His, Asn266 into Met or Asn266 into Trp, showed an improved conversion of CPC compared to the activity of the wild-type enzyme. Variants carrying mutations Asn266 into Gln or Asn266 into His displayed almost a two-fold improved conversion of cephalosporin C over wild-type, whereas the variant carrying mutation Asn266 into Met showed an improvement of about 30% (Fig. 2). Hence, this example clearly shows that the variants according to the present inventions are indeed very effective enzymes for the conversion of cephalosporin C into 7-ACA.
Similarly, the variant carrying mutation Tyr178 into His was shown to be 3-fold more active towards cephalosporin C (Table 2).

### Example 7 - Some multiple mutated variants of Pseudomonas SY-77 glutaryl amidase with increased cephalosporin C hydrolysis activity

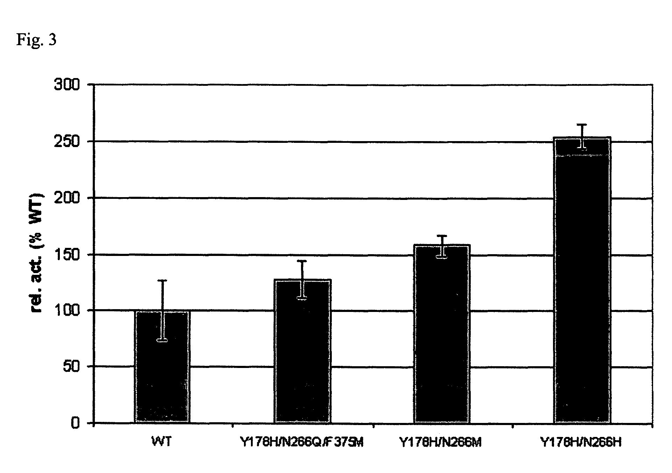

The single mutants of the glutaryl amidase from *Pseudomonas* SY-77 comprising mutations Asn266 into glutamine, Asn266 into histidine or Asn266 into methionine were combined with different other mutations. Some mutants were purified and the hydrolysis activity towards cephalosporin C acylase was determined. The conversion rate of the different mutants was calculated from an assay with 70 µg enzyme. The mean slope of the conversion of cephalosporin C in the first 4 hours was determined and normalised towards WT (see Figure 1). The multiple mutants SY-77^{N266H+Y178H}, SY-77^{N266M+Y178H} and SY-77^{N266Q+Y178H+F375M} appeared to have a higher hydrolysis activity towards cephalosporin C than the wild-type glutaryl amidase (Fig 3).

### Example 8 - Some single mutated variants of Pseudomonas SY-77 glutaryl amidase carrying a mutation at position Asn266 with increased hydrolysis activity towards adipyl-7-ADCA

Wild-type (WT) and 15 glutaryl amidases variants mutated at position 266 were produced in *E. coli* DH10B and purified up to 95% purity. The 4 glutaryl amidases comprising single mutations N266R, N266I, N266K and N266V could not be produced. The catalytic parameters of the remaining 16 enzymes were determined measuring the initial rate of hydrolysis on a range of substrate concentrations with a fixed amount of enzyme using the Multiprobe II for all pipetting steps. 140 µl 20 mM phosphate buffer pH 7.5 with substrate (0.06-2 mM glutaryl-7-ACA or 0.2-10 mM adipyl-7-ADCA) was preheated at 37°C. 40µl phosphate buffer containing an appropriate amount of purified enzyme was added, starting the reaction. After 10 min incubation at 37°C 40 µl 2.5 M acetate buffer pH 4.5 was added to stop the reaction. 20 µl 1 mg/ml fluorescamine in acetone was added and the A380 was measured after 60 min incubation at room temperature.
Kinetic parameters were obtained by fitting the experimental data from Eadie-Hofstee plots, and the mean and standard deviation of values of at least 4 independent measurements were calculated. The k_{cat} was calculated using the theoretical molecular weight of the mature enzyme, 75.9 kDa. Variants carrying mutations Asn266 into Met, His or Gln displayed a 2- to 15-fold improved catalytic efficiency over wild-type according to the hydrolysis of adipyl-7-ADCA, whereas the variants carrying mutation Asn266 into Phe, Trp, Tyr or Cys were equally or 150% as efficient as WT in the hydrolysis of adipyl-7-ADCA. Hence, this example clearly shows that the variants according to the present inventions are indeed very effective enzymes for the conversion of adipyl-7-ADCA into 7-ADCA.

### Example 9 - Some multiple mutated variants of Pseudomonas SY-77 glutaryl amidase selected on adipyl-leucine with increased hydrolysis activity towards adipyl-7-ADCA

Two libraries of multiple mutated glutaryl amidase of *Pseudomonas* SY-77 were constructed. The first one comprised all randomized codons at positions Asn266 and Phe375 as described in example 3. In the second library, the Tyr178 of all the plasmids of library 1, were replaced by a His. *E. coli* DH10B cells containing the mutant plasmids were slowly thawed on ice and washed twice with 0.9% NaCl. They were starved by incubation in 0.9% NaCl for 2 h at 37°C and plated onto selective minimal medium plates containing 0.1 mg/ml adipyl-leucine as a sole leucine source. Approximately 5000 viable *E. coli* DH10B cells containing the mutant plasmids were spread onto each plate. Of each library a total of 10⁵ transformants was plated. Plates were incubated at 30°C for at least 10 days. Every day new appearing colonies were marked. Colonies were selected on the basis of their date of appearance and size, and streaked again on minimal medium containing adipyl-leucine as sole leucine source to ensure unique colonies were obtained. Single colonies from these second plates were used to inoculate an overnight culture to determine acylase activity and to isolate plasmid DNA for sequence determination. Four colonies with unique sequences appeared to have an improved ratio of adipyl-7-ADCA hydrolysis over glutaryl-7-ACA hydrolysis. These mutants were purified and their catalytic parameters on glutaryl-7-ACA and adipyl-7-ADCA were determined as described in example 8. Furthermore, the hydrolysis of cephalosporin C was measured as described in example 2. The hydrolytic properties of these enzymes towards these different substrates are listed in table 2. It is obvious that all measured mutants have a better catalytic efficiency towards adipyl-7-ADCA mostly due to a lowered Kₘ. Furthermore, 3 out of the 4 mutants tested have an improved hydrolysis rate of cephalosporin C. It is not clear if this activity stems from an improved k_{cat} and/or Kₘ. This example shows, however, that variants of the glutaryl amidase with an improved activity towards adipyl-7-ADCA can be isolated using the described selection method.

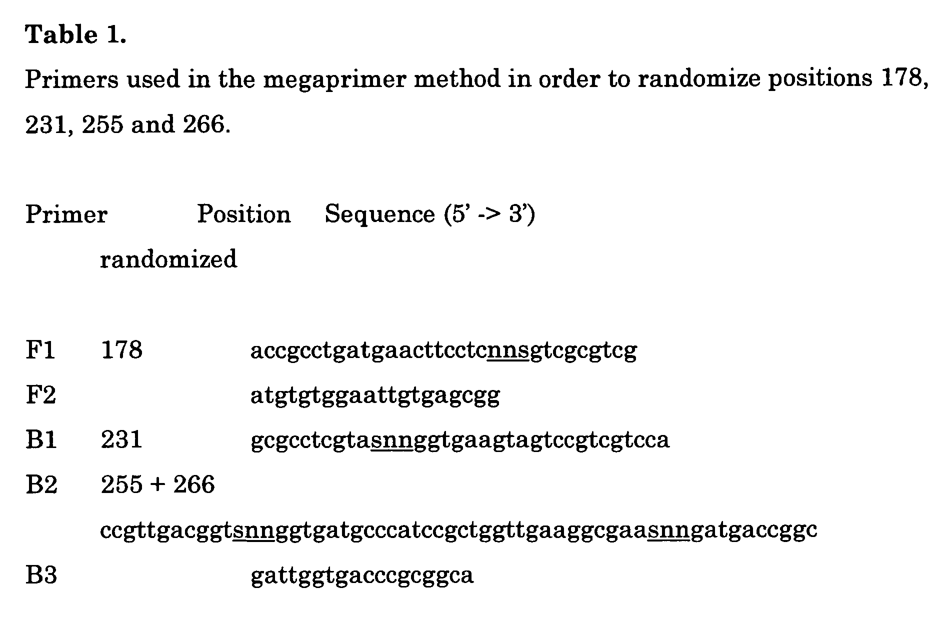

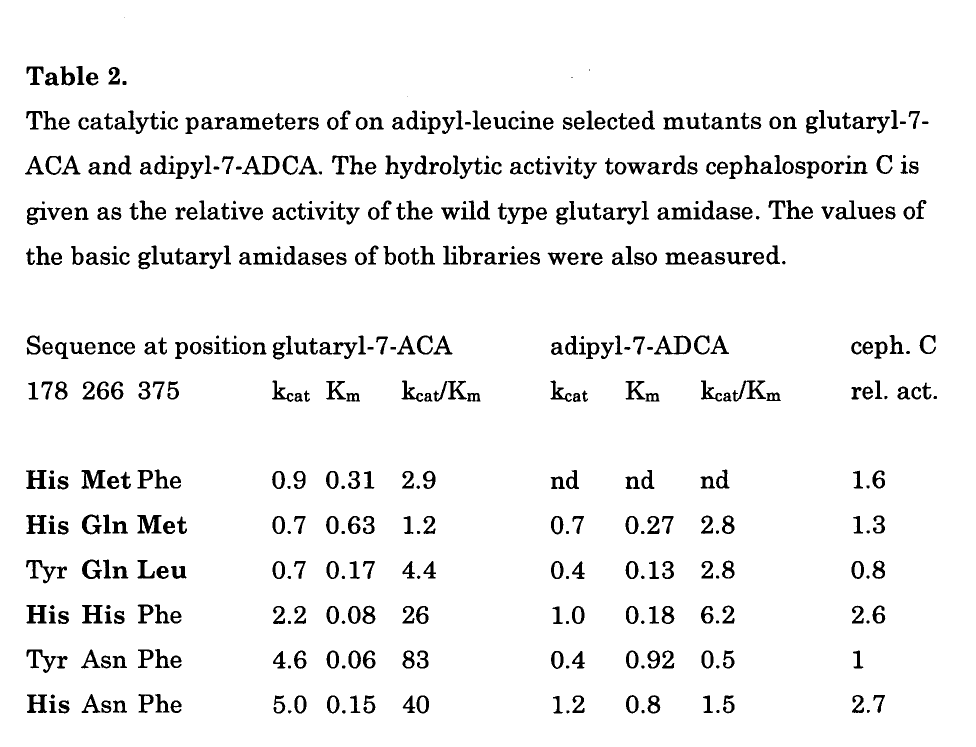

### References

1. Yamada, H., Ishii, Y., Noguchi, Y., Miura, T., Mori, T., and Saito, Y. (1996) Protein engineering of a cephalosporin C acylase. Ann. N. Y. Acad. Sci. 799, 74-81.
2. Ishii, Y., Saito, Y., Fujimura, T., Sasaki, H., Noguchi, Y., Yamada, H., Niwa, M., and Shimomura, K. (1995) High-level production, chemical modification and site-directed mutagenesis of a cephalosporin C acylase from Pseudomonas strain N176. Eur. J. Biochem. 230, 773-778.
3. Saito, Y., Fujimura, T., Ishii, Y., Noguchi, Y., Miura, T., Niwa, M., and Shimomura, K. (1996) Oxidative modification of a cephalosporin C acylase from Pseudomonas strain N176 and site-directed mutagenesis of the gene. Appl. Environ. Microbiol. 62, 2919-2925.
4. Saito, Y., Ishii, Y., Fujimura, T., Sasaki, H., Noguchi, Y., Yamada, H., Niwa, M., and Shimomura, K. (1996) Protein engineering of a cephalosporin C acylase from Pseudomonas strain N176. Ann. N. Y. Acad. Sci. 782, 226-240.
5. Fritz-Wolf, K. Koller, K.P., Lange, G., Liesum, A., Sauber, K., Schreuder, H., Aretz, W., and Kabsch, W. (2002) Protein Sci. 11, 92-103
6. Sio, C.F., Riemens, A.M., van der Laan, J.M., Verhaert, R.M.D., and Quax, W.J. (2002) Directed evolution of a glutaryl acylase into an adipyl acylase. Eur. J. Biochem. 269, 4495-4504.
7. Elander R.P. (2003) Appl. Microbiol. Biotechnol. 61, 385-392
8. Oh B, Kim M, Yoon J, Chung K, Shin Y, Lee D, Kim Y. (2003) Deacylation activity of cephalosporin acylase to cephalosporin C is improved by changing the side-chain conformations of active-site residues. Biochem Biophys Res Commun. 10;310(1):19-27.
9. Otten, L.G., Sio, C.F., Vrielink, J. Cool, R.H., and Quax, W.J. (2002) J. Biol. Chem. 277 (44), 42121-42127
10. Ishii, Y., Saito, Y., Fujimura, T., Isogai, T., Kojo, H., Yamashita, M., Niwa, M., and Kohsaka, M. (1994) A Novel 7-ß-(4-carboxybutanamido)-cephalosporanic acid acylase isolated from Pseudomonas strain C427 and its high-level production in Escherichia coli. Journal of Fermentation and Bioengineering. 77, 591-597.
11. Li, Y., Chen, J., Jiang, W., Mao, X., Zhao, G., and Wang, E. (1999) In vivo post-translational processing and subunit reconstitution of cephalosporin acylase from Pseudomonas sp. 130. Eur. J. Biochem. 262, 713-719.
12. Li, Y., Jiang, W., Yang, Y., Zhao, G., and Wang, E. (1998) Overproduction and purification of glutaryl 7-amino cephalosporanic acid acylase. Protein Expr. Purif. 12, 233-238.
13. Ichikawa, S., Murai, Y., Yamamoto, S., Shibuya, Y., Fujii, T., Komatsu, K., and Kodaira, R. (1981) The isolation and properties of Pseudomonas mutants with an enhanced productivity of 7-□-(4-carboxybutanamido)-cephalosporanic acid acylase. Agric. Biol. Chem. 45, 2225-2229.
14. Matsuda, A. and Komatsu, K.I. (1985) Molecular cloning and structure of the gene for 7-ß-(4-carboxybutanamido)cephalosporanic acid acylase from a Pseudomonas strain. J. Bacteriol. 163, 1222-1228.
15. Kim, D.W., Kang, S.M., and Yoon, K.H. (1999) Isolation of novel Pseudomonas diminuta KAC-1 strain producing glutaryl 7-aminocephalosporanic acid acylase. J. Microbiol. 37, 200-205.
16. Kim, Y., Yoon, K., Khang, Y., Turley, S., and Hol, W.G. (2000) The 2.0 Å crystal structure of cephalosporin acylase. Structure 8, 1059-1068.
17. Shibuya, Y., Matsumoto, K., and Fujii, T. (1981) Isolation and properties of 7-□-(4-carboxybutanamido)cephalosporanic acid acylase-producing bacteria. Agric. Biol. Chem. 45, 1561-1567.
18. Verhaert et al, 1997, Appl Environ Microbiol. 1997 Sep;63(9):3412-8.
19. Biokatalysatoren und Enzymtechnologie, K. Buchholz and V. Kasche, VCH Verlag, Weinheim, 1st edition 1997
20. Cao, L., Van Langen, L., and Sheldon, R.A. (2003) Immobilised enzymes: carrier-bound or carrier-free? Curr. Op. Biotechnol. 14, 387-394.
21. Bruggink, A., Roos E.C., de Vroom E., 1998) Organic Process Res Dev. 2, 128-133
22. Ausubel, F. et al, Current Protocols in Molecular Biology, Greene Publishing Associates and Wiley Interscience, 1994-1998 and Supplements.
23. Landt, O., Grunert, H.P., and Hahn, U. (1990) Gene 1990, 96 125-128.
24. Reyes F, Martinez M.J., and Soliveri J. (1989) Determination of cephalosporin-C amidohydrolase activity with fluorescamine. J Pharm Pharmacol. 41(2):136-7.

## Claims

1. An isolated or single mutated glutaryl amidase from *Pseudomonas* SY-77, or a functional part, derivative or analogue thereof, having an amino acid at position 266 other than asparagine, histidine or serine.

2. A glutaryl amidase according to claim 1, having a glutamine, methionine or tryptophan at position 266.

3. A glutaryl amidase according to claim 2, having a glutamine or methionine at position 266.

4. A glutaryl amidase according to claim 3, having a glutamine at position 266.

5. An isolated or multiple mutated glutaryl amidase from *Pseudomonas* SY-77, or a functional part, derivative or analogue thereof, having an amino acid other than its native amino at position Asn266, and having an amino acid other than its native amino acid at one or more positions Leu177, Tyr178, Val179, His221, Leu222, Phe229, Tyr231, Gln248, Arg255, Phe256, Thr267, Val268, Met271, Gln291, Met347, Tyr351, Thr374, Phe375 and/or Asn442.

6. A glutaryl amidase according to claim 5, having an amino acid other than its native amino acid at one or more positions Tyr178, Leu222, Tyr231, Gln248, Arg255, Val268, Tyr351 and/or Phe375.

7. A glutaryl amidase according to claim 5 or 6, having an amino acid other than its native amino acid at one or more of the following positions:
Tyr178,
Tyr231,
Arg255,
Phe375,
Tyr178 + Tyr231,
Tyr178 + Arg255,
Tyr178 + Phe375,
Tyr231 + Arg255,
Tyr231 + Phe375,
Tyr231 + Arg255 + Phe375,
Arg255 + Phe375,
Tyr178 + Tyr231 + Arg255,
Tyr178 + Tyr231 + Phe375,
Tyr178 + Arg255 + Phe375, or
Tyr178 + Tyr231 + Arg255 + Phe375.

8. A glutaryl amidase according to any one of claims 5-7, having a histidine, glutamine, methionine or tryptophan at position 266.

9. A glutaryl amidase according to claim 8, having a histidine, glutamine or methionine at position 266.

10. A glut aryl amidase according to claim 9, having a glutamine at position 266.

11. A polynucleotide encoding a glutaryl amidase according to any one of claims 1-10.

12. A nucleic acid vector comprising a polynucleotide according to claim 11.

13. A host cell comprising a polynucleotide according to claim 11, which polynucleotide is operably linked to a regulatory control sequence which allow for direct expression of the variant of a glutaryl amidase in said host cell.

14. A method for producing a glutaryl amidase according to any one of claims 1 to 10, which method comprises culturing a host cell according to claim 13 under suitable conditions which allow for expression of the glutaryl amidase in said host cell.

15. A method for preparing 7-aminocephalosporanic acid from cephalosporin C, which method comprises reacting cephalosporin C with an isolated, single mutated or multiple mutated glutaryl amidase from *Pseudomonas* SY-77, or a functional part, derivative or analogue thereof, having an amino acid at position 266 other than asparagine under suitable conditions such that the glutaryl amidase cleaves the cephalosporin C directly to form the 7-aminocephalosporanic acid.

16. A method for preparing 7-aminocephalosporanic acid from cephalosporin C, which method comprises reacting cephalosporin C with a variant of a glutaryl amidase according to any one of claims 1-10 under suitable conditions such that the glutaryl amidase cleaves the cephalosporin C directly to form the 7-aminocephalosporanic acid.

17. A method for preparing 7-aminocephalosporanic acid from cephalosporin C, which method comprises reacting cephalosporin C with an isolated, single or multiple mutated glutaryl amidase from *Pseudomonas* SY-77, or a functional part, derivative or analogue thereof, having an amino acid other than its native amino acid at position Tyr178, under suitable conditions such that the glutaryl amidase cleaves the cephalosporin C directly to form the 7-aminocephalosporanic acid.

18. A method according to claim 17, having a histidine at position Tyr178.

19. A method for preparing 7-aminocephalosporanic acid from cephalosporin C according to claim 16, wherein the glutaryl amidase is a single mutated glutaryl amidase.

20. A method for preparing aminodesacetoxycephalosporanic acid, which comprises reacting adipyl-7-ADCA with an isolated or single mutated glutaryl amidase from *Pseudomonas* SY-77, or a functional part, derivative or analogue thereof, having an amino acid other than its native amino acid at position Asn266, under suitable conditions such that the glutaryl amidase cleaves adipyl-7-ADCA to form the aminodesacetoxycephalosporanic acid.

21. A method for developing a glutaryl amidase with cephalosporin C hydrolysis activity comprising the steps of:
1) applying a mutagenesis technique in order to create a large library of mutants of a glutaryl amidase according to the present invention that carries a mutation in one or more positions; and subsequently
2) applying a selection procedure comprising growth of proper host cells that express variants of said glutaryl amidase on solid medium containing a selective, aminoadipyl-containing compound and identification of variants with the desired enzymatic activity.

22. A method for the preparation of 7-ACA based antibiotics comprising incubating 7-ACA in the presence of a glutaryl amidase according to any one of claims 1-10 with alkyl side chains under suitable synthesizing conditions.


SEQUENCE LISTING

<110>  Stichting voor de Technische Wetenschappen

<120>  Glutaryl amidases and their uses

<130>  P67064EP00

<140>  EP 03078861.6
<141>  2003-12-05

<160>  9

<170>  PatentIn version 3.1

<210>  1
<211>  32
<212>  DNA
<213>  Artificial Sequence

<220>
<223>  Primer F1
<220>
<221>  misc_feature
<222>  (21)..(22)
<223>  "n" on pos. 21 and 22 stands for unknown nucleic acid

<400>  1
accgcctgat gaacttcctc nnsgtcgcgt cg                                    32

<210>  2
<211>  20

<212>  DNA

<213>  Artificial Sequence



<220>

<223>  Primer F2

<400>  2
atgtgtggaa ttgtgagcgg                                                    20


<210>  3

<211>  34

<212>  DNA

<213>  Artificial Sequence



<220>

<223>  Primer B1

<220>

<221>  misc_feature

<222>  (12)..(13)

<223>  "n" on pos. 12 and 13 stands for unknown nucleic acid



<400>  3
gcgcctcgta snnggtgaag tagtccgtcg tcca                                    34


<210>  4

<211>  57

<212>  DNA

<213>  Artificial Sequence



<220>

<223>  Primer B2

<220>

<221>  misc_feature

<222>  (13)..(14)

<223>  "n" on pos. 13 and 14 stands for unknown nucleic acid

<220>

<221>  misc_feature

<222>  (46)..(47)

<223>  "n" on pos. 46 and 47 stands for unknown nucleic acid

<400>  4
ccgttgacgg tsnnggtgat gcccatccgc tggttgaagg cgaasnngat gaccggc        57

<210>  5

<211>  18

<212>  DNA

<213>  Artificial Sequence

<220>

<223>  Primer B3

<400>  5
gattggtgac ccgcggca                                                   18

<210>  6

<211>  2163

<212>  DNA

<213>  Pseudomonas SY-77

<220>

<221>  misc_feature

<222>  (1)..(2161)

<223>  glutaryl amidase

<400>  6
atgctgagag ttctgcaccg ggcggcgtcc gccttggtta tggcgactgt gatcggcctt      60

gcgcccgccg tcgcctttgc gctggccgag ccgacctcga cgccgcaggc gccgattgcg 120
gcctataaac cgagaagcaa tgagatcctg tgggacggct acggcgtccc gcacatctac 180
ggcgtcgacg cgccctcagc cttctacggc tatggctggg cccaggcgcg cagccacggc 240
gacaatatcc tgcgcctgta tggagaagcg cggggcaagg gggccgaata ctggggcccg 300
gattacgaac agacgaccgt ctggctgctg accaacggcg tgccggagcg cgctcagcag 360
tggtatgcgc agcagtcgcc tgatttccgc gccaacctcg acgccttcgc ggcgggcatc 420
aacgcctatg cgcagcagaa ccccgacgac atctcgcccg aggtgcggca ggtgctgccg 480
gtttccggcg ccgacgtggt ggcccacgcc caccgcctga tgaacttcct ctatgtcgcg 540
tcgcccggcc gcaccctggg cgagggcgac ccgccggacc tggccgatca aggatccaac 600
tcctgggcgg tggcgccggg aaagacggcg aacgggaacg ccctgctgct gcagaacccg 660
cacctgtcct ggacgacgga ctacttcacc tactacgagg cgcatctcgt cacgccggac 720
ttcgaaatct atggcgcgac ccagatcggc ctgccggtca tccgcttcgc cttcaaccag 780
cggatgggca tcaccaatac cgtcaacggc atggtggggg ccaccaacta tcggctgacg 840
cttcaggacg gcggctatct gtatgacggt caggtgcggc cgttcgagcg gcctcaggcc 900
tcgtatcgcc tgcgtcaggc ggacgggacg acggtcgaca agccgttgga gatccgctcc 960
agcgtccatg gcccggtctt cgagcgcgcg gacggcacgg ccgtcgccgt tcgggtcgcc 1020
ggtctggacc ggccgggcat gctcgagcag tatttcgaca tgatcacggc ggacagcttc 1080
gacgactacg aagccgcttt ggcgcggatg caggtgccga ccttcaacat cgtctacgcc 1140
gaccgcgaag ggaccatcaa ctacagcttc aacggcgtgg cgcccaaacg ggccgagggc 1200
gacatcgcct tctggcaggg gctcgtgccg ggcgattcct cgcgttacct gtggaccgag 1260
acacacccgc tggacgatct gccgcgcgtc accaatccgc cgggcggctt cgtgcagaac 1320
tccaatgatc cgccgtggac gccgacctgg cccgtcacct acacgcccaa ggacttcccc 1380
tcctatctgg cgccccagac gccgcattcc ctgcgtgcgc aacaaagcgt gcgtctgatg 1440
tccgagaacg acgacctgac gctggagcgc ttcatggcgc tgcagttgag ccatcgcgcc 1500
gtcatggccg accgcacctt gccggacctg atcccggccg ccctgatcga ccccgatccc 1560
gaggtccagg cggcggcgcg cctgctggcg gcgtgggatc gcgagttcac cagcgacagc 1620
cgcgccgccc tgctgttcga ggaatgggcg cgtctgttcg ccggccagaa tttcgcaggc 1680
caggccggct tcgccacgcc ctggtcgctg gataagccgg tcagcacgcc ttacggcgtc 1740
cgcgacccca aggccgccgt cgatcaactg cggaccgcca tcgccaacac caagcgcaaa 1800
tacggcgcga tcgaccggcc gttcggcgac gcctcgcgca tgatcctgaa cgacgtgaat 1860
gttccgggcg ccgccggcta cggcaacctg ggttccttcc gggtcttcac ctggtccgat 1920

cctgacgaaa acggggttcg cacgcccgtc cacggcgaga cgtgggtggc gatgatcgag 1980

ttctccacgc cggtgcgggc ctatggcctg atgagctacg gcaactctcg ccagccgggc 2040

acgacgcact acagcgatca gatcgaacgc gtgtcgcgcg ccgacttccg cgaactgttg 2100

ctgcggcgag agcaggtcga ggccgccgtc caggaacgca cgcccttcaa cttcaagcca 2160

tga 2163

<210> 7

<211> 720

<212> PRT

<213> Pseudomonas SY-77

<220>

<221> SITE

<222> (1)..(720)

<223> glutaryl amidase propeptide

<400> 7

Met Leu Arg Val Leu His Arg Ala Ala Ser Ala Leu Val Met Ala Thr
1               5                   10                  15

Val Ile Gly Leu Ala Pro Ala Val Ala Phe Ala Leu Ala Glu Pro Thr
            20                  25                  30

Ser Thr Pro Gln Ala Pro Ile Ala Ala Tyr Lys Pro Arg Ser Asn Glu
        35                  40                  45

Ile Leu Trp Asp Gly Tyr Gly Val Pro His Ile Tyr Gly Val Asp Ala
    50                  55                  60

Pro Ser Ala Phe Tyr Gly Tyr Gly Trp Ala Gln Ala Arg Ser His Gly
65                  70                  75                  80

Asp Asn Ile Leu Arg Leu Tyr Gly Glu Ala Arg Gly Lys Gly Ala Glu
                85                  90                  95

Tyr Trp Gly Pro Asp Tyr Glu Gln Thr Thr Val Trp Leu Leu Thr Asn
            100                 105                 110

Gly Val Pro Glu Arg Ala Gln Gln Trp Tyr Ala Gln Gln Ser Pro Asp
        115                 120                 125

Phe Arg Ala Asn Leu Asp Ala Phe Ala Ala Gly Ile Asn Ala Tyr Ala
    130                 135                 140

Gln Gln Asn Pro Asp Asp Ile Ser Pro Glu Val Arg Gln Val Leu Pro
145                 150                 155                 160

Val Ser Gly Ala Asp Val Val Ala His Ala His Arg Leu Met Asn Phe
                165                 170                 175

Leu Tyr Val Ala Ser Pro Gly Arg Thr Leu Gly Glu Gly Asp Pro Pro
            180                 185                 190

Asp Leu Ala Asp Gln Gly Ser Asn Ser Trp Ala Val Ala Pro Gly Lys
        195                 200                 205

Thr Ala Asn Gly Asn Ala Leu Leu Leu Gln Asn Pro His Leu Ser Trp
    210                 215                 220

Thr Thr Asp Tyr Phe Thr Tyr Tyr Glu Ala His Leu Val Thr Pro Asp
225                 230                 235                 240

Phe Glu Ile Tyr Gly Ala Thr Gln Ile Gly Leu Pro Val Ile Arg Phe
                245                 250                 255

Ala Phe Asn Gln Arg Met Gly Ile Thr Asn Thr Val Asn Gly Met Val
            260                 265                 270

Gly Ala Thr Asn Tyr Arg Leu Thr Leu Gln Asp Gly Gly Tyr Leu Tyr
        275                 280                 285

Asp Gly Gln Val Arg Pro Phe Glu Arg Pro Gln Ala Ser Tyr Arg Leu
    290                 295                 300

Arg Gln Ala Asp Gly Thr Thr Val Asp Lys Pro Leu Glu Ile Arg Ser
305                 310                 315                 320

Ser Val His Gly Pro Val Phe Glu Arg Ala Asp Gly Thr Ala Val Ala
                325                 330                 335

Val Arg Val Ala Gly Leu Asp Arg Pro Gly Met Leu Glu Gln Tyr Phe
            340                 345                 350

Asp Met Ile Thr Ala Asp Ser Phe Asp Asp Tyr Glu Ala Ala Leu Ala
        355                 360                 365

Arg Met Gln Val Pro Thr Phe Asn Ile Val Tyr Ala Asp Arg Glu Gly
    370                 375                 380

Thr Ile Asn Tyr Ser Phe Asn Gly Val Ala Pro Lys Arg Ala Glu Gly
385                 390                 395                 400

Asp Ile Ala Phe Trp Gln Gly Leu Val Pro Gly Asp Ser Ser Arg Tyr
                405                 410                 415

Leu Trp Thr Glu Thr His Pro Leu Asp Asp Leu Pro Arg Val Thr Asn
            420                 425                 430

Pro Pro Gly Gly Phe Val Gln Asn Ser Asn Asp Pro Pro Trp Thr Pro
        435                 440                 445

Thr Trp Pro Val Thr Tyr Thr Pro Lys Asp Phe Pro Ser Tyr Leu Ala
    450                 455                 460

Pro Gln Thr Pro His Ser Leu Arg Ala Gln Gln Ser Val Arg Leu Met
465                 470                 475                 480

Ser Glu Asn Asp Asp Leu Thr Leu Glu Arg Phe Met Ala Leu Gln Leu
                485                 490                 495

Ser His Arg Ala Val Met Ala Asp Arg Thr Leu Pro Asp Leu Ile Pro
            500                 505                 510

Ala Ala Leu Ile Asp Pro Asp Pro Glu Val Gln Ala Ala Ala Arg Leu
        515                 520                 525

Leu Ala Ala Trp Asp Arg Glu Phe Thr Ser Asp Ser Arg Ala Ala Leu
    530                 535                 540

Leu Phe Glu Glu Trp Ala Arg Leu Phe Ala Gly Gln Asn Phe Ala Gly
545                 550                 555                 560

Gln Ala Gly Phe Ala Thr Pro Trp Ser Leu Asp Lys Pro Val Ser Thr
                565                 570                 575

Pro Tyr Gly Val Arg Asp Pro Lys Ala Ala Val Asp Gln Leu Arg Thr
            580                 585                 590

Ala Ile Ala Asn Thr Lys Arg Lys Tyr Gly Ala Ile Asp Arg Pro Phe

595                 600                 605

Gly Asp Ala Ser Arg Met Ile Leu Asn Asp Val Asn Val Pro Gly Ala
    610                 615                 620

Ala Gly Tyr Gly Asn Leu Gly Ser Phe Arg Val Phe Thr Trp Ser Asp
625                 630                 635                 640

Pro Asp Glu Asn Gly Val Arg Thr Pro Val His Gly Glu Thr Trp Val
                645                 650                 655

Ala Met Ile Glu Phe Ser Thr Pro Val Arg Ala Tyr Gly Leu Met Ser
            660                 665                 670

Tyr Gly Asn Ser Arg Gln Pro Gly Thr Thr His Tyr Ser Asp Gln Ile
        675                 680                 685

Glu Arg Val Ser Arg Ala Asp Phe Arg Glu Leu Leu Leu Arg Arg Glu
    690                 695                 700

Gln Val Glu Ala Ala Val Gln Glu Arg Thr Pro Phe Asn Phe Lys Pro
705                 710                 715                 720

<210>  8

<211>  161

<212>  PRT

<213>  Pseudomonas SY-77

<220>

<221>  SITE

<222>  (1)..(161)

<223>  glutaryl amidase A chain (pos. 28 - 188)

<400>  8

Leu Ala Glu Pro Thr Ser Thr Pro Gln Ala Pro Ile Ala Ala Tyr Lys
1               5                   10                  15

Pro Arg Ser Asn Glu Ile Leu Trp Asp Gly Tyr Gly Val Pro His Ile
            20                  25                  30

Tyr Gly Val Asp Ala Pro Ser Ala Phe Tyr Gly Tyr Gly Trp Ala Gln
        35                  40                  45

Ala Arg Ser His Gly Asp Asn Ile Leu Arg Leu Tyr Gly Glu Ala Arg
    50                  55                  60

Gly Lys Gly Ala Glu Tyr Trp Gly Pro Asp Tyr Glu Gln Thr Thr Val
65                  70                  75                  80

Trp Leu Leu Thr Asn Gly Val Pro Glu Arg Ala Gln Gln Trp Tyr Ala
                85                  90                  95

Gln Gln Ser Pro Asp Phe Arg Ala Asn Leu Asp Ala Phe Ala Ala Gly
            100                 105                 110

Ile Asn Ala Tyr Ala Gln Gln Asn Pro Asp Asp Ile Ser Pro Glu Val
        115                 120                 125

Arg Gln Val Leu Pro Val Ser Gly Ala Asp Val Val Ala His Ala His
    130                 135                 140

Arg Leu Met Asn Phe Leu Tyr Val Ala Ser Pro Gly Arg Thr Leu Gly
145                 150                 155                 160

Glu

<210>  9

<211>  522

<212>  PRT

<213>  Pseudomonas SY-77

<220>

<221>  SITE

<222>  (1)..(522)

<223>  glutaryl amidase B chain (pos. 199 - 720)

<400>  9

Ser Asn Ser Trp Ala Val Ala Pro Gly Lys Thr Ala Asn Gly Asn Ala
1               5                   10                  15

Leu Leu Leu Gln Asn Pro His Leu Ser Trp Thr Thr Asp Tyr Phe Thr
            20                  25                  30

Tyr Tyr Glu Ala His Leu Val Thr Pro Asp Phe Glu Ile Tyr Gly Ala
        35                  40                  45

Thr Gln Ile Gly Leu Pro Val Ile Arg Phe Ala Phe Asn Gln Arg Met
    50                  55                  60

Gly Ile Thr Asn Thr Val Asn Gly Met Val Gly Ala Thr Asn Tyr Arg
65                  70                  75                  80

Leu Thr Leu Gln Asp Gly Gly Tyr Leu Tyr Asp Gly Gln Val Arg Pro
                85                  90                  95

Phe Glu Arg Pro Gln Ala Ser Tyr Arg Leu Arg Gln Ala Asp Gly Thr
            100                 105                 110

Thr Val Asp Lys Pro Leu Glu Ile Arg Ser Ser Val His Gly Pro Val
        115                 120                 125

Phe Glu Arg Ala Asp Gly Thr Ala Val Ala Val Arg Val Ala Gly Leu
    130                 135                 140

Asp Arg Pro Gly Met Leu Glu Gln Tyr Phe Asp Met Ile Thr Ala Asp
145                 150                 155                 160

Ser Phe Asp Asp Tyr Glu Ala Ala Leu Ala Arg Met Gln Val Pro Thr
                165                 170                 175

Phe Asn Ile Val Tyr Ala Asp Arg Glu Gly Thr Ile Asn Tyr Ser Phe
            180                 185                 190

Asn Gly Val Ala Pro Lys Arg Ala Glu Gly Asp Ile Ala Phe Trp Gln
        195                 200                 205

Gly Leu Val Pro Gly Asp Ser Ser Arg Tyr Leu Trp Thr Glu Thr His
    210                 215                 220

Pro Leu Asp Asp Leu Pro Arg Val Thr Asn Pro Pro Gly Gly Phe Val
225                 230                 235                 240

Gln Asn Ser Asn Asp Pro Pro Trp Thr Pro Thr Trp Pro Val Thr Tyr
                245                 250                 255

Thr Pro Lys Asp Phe Pro Ser Tyr Leu Ala Pro Gln Thr Pro His Ser
            260                 265                 270

Leu Arg Ala Gln Gln Ser Val Arg Leu Met Ser Glu Asn Asp Asp Leu
        275                 280                 285

Thr Leu Glu Arg Phe Met Ala Leu Gln Leu Ser His Arg Ala Val Met
    290                 295                 300

Ala Asp Arg Thr Leu Pro Asp Leu Ile Pro Ala Ala Leu Ile Asp Pro
305                 310                 315                 320

Asp Pro Glu Val Gln Ala Ala Ala Arg Leu Leu Ala Ala Trp Asp Arg
                325                 330                 335

Glu Phe Thr Ser Asp Ser Arg Ala Ala Leu Leu Phe Glu Glu Trp Ala
            340                 345                 350

Arg Leu Phe Ala Gly Gln Asn Phe Ala Gly Gln Ala Gly Phe Ala Thr
        355                 360                 365

Pro Trp Ser Leu Asp Lys Pro Val Ser Thr Pro Tyr Gly Val Arg Asp
    370                 375                 380

Pro Lys Ala Ala Val Asp Gln Leu Arg Thr Ala Ile Ala Asn Thr Lys
385                 390                 395                 400

Arg Lys Tyr Gly Ala Ile Asp Arg Pro Phe Gly Asp Ala Ser Arg Met
                405                 410                 415

Ile Leu Asn Asp Val Asn Val Pro Gly Ala Ala Gly Tyr Gly Asn Leu
            420                 425                 430

Gly Ser Phe Arg Val Phe Thr Trp Ser Asp Pro Asp Glu Asn Gly Val
        435                 440                 445

Arg Thr Pro Val His Gly Glu Thr Trp Val Ala Met Ile Glu Phe Ser
    450                 455                 460

Thr Pro Val Arg Ala Tyr Gly Leu Met Ser Tyr Gly Asn Ser Arg Gln
465                 470                 475                 480

Pro Gly Thr Thr His Tyr Ser Asp Gln Ile Glu Arg Val Ser Arg Ala
                485                 490                 495

Asp Phe Arg Glu Leu Leu Leu Arg Arg Glu Gln Val Glu Ala Ala Val

            500                 505                 510

Gln Glu Arg Thr Pro Phe Asn Phe Lys Pro
        515                 520